# EUROPEAN PATENT APPLICATION

(11) **EP 1 484 020 A1**
(43) Date of publication of application: **08.12.2004**
(21) Application number: 03012948.0
(22) Date of filing: 06.06.2003
(51) Int. Cl.: A61B 8/12, G01S 15/89

(54) **Motorized multiplane transesophageal probe with coupling fluid**

(71) Applicant: Kontron Medical AG, 4053 Basel (CH)
(72) Inventor: Liard, Marc, 4153 Reinach / BL (CH); Schlaepfer, Claude, 4461 Böchten / BL (CH); Welter, André, 68870 Bartenheim (FR); Kissling, Beat, 4153 Reinach (CH)
(74) Representative: Braun, André

(57) **Abstract**

A multiplane ultrasound probe, particularly for echocardiographic use, has a phased array transducer and a motor for rotating the transducer in its tip. A liquid film is provided between the acoustic window and the transducer. A spring presses the transducer against the film and the acoustic window. Thus acoustic coupling and a sliding bearing effect is achieved.

## Description

This invention relates to multiplane ultrasound probes with a probe tip containing a phased array transducer and a motor for rotating the transducer array. According to a more particular aspect the invention relates to transesophageal probes for echocardiography.

Ultrasound imaging of the heart (also called echocardiography) is usually performed with an ultrasound probe from outside the body. The ultrasound pulses have to pass different structures between the body surface and the heart which influence the echo data. The image quality is therefore often decreased. Instead of applying a transthoracic ultrasound probe, a transesophageal probe can be used. By placing the ultrasound probe inside the esophagus, the distance from the transducer to the heart and also the intermediate structures can be reduced resulting in better imaging performance.

Such a transesophageal probe consists of an ultrasound transducer mounted at the distal end of an endoscope. The ultrasound transducer is normally a phased array which can scan a two-dimensional imaging plane. In order to be able to image different cross-sections of the heart, the transducer is mounted on a mechanism which allows the transducer to be rotated around its center axis.

The rotation can be controlled manually by a knob at the proximal handle of the endoscope. The rotation of the control knob is transferred to the rotation mechanism of the transducer by a flexible wire guide system within the endoscope. The manual rotation knob at the handle of the endoscope can be replaced by a motor and a corresponding electronic control system which improves the reproducibility of the scan plane positioning. Nevertheless, due to the mechanical lag in the mechanical wire guide system through the whole length of the endoscope, the accuracy of the scan plane angle is marginal.

Three-dimensional volumic structures can be reconstructed from two-dimensional images scanned at differently oriented planes. The accuracy of the positional information of the different images influence essentially the quality of the 3D reconstruction and mainly the achievable precision of volume measurements.

An improvement in the accuracy for the positioning of the scan plane has been achieved by placing a micro-motor directly aside the ultrasound transducer at the distal end of the endoscope.

The ultrasound transducer has to be coupled acoustically to the body through acoustically matched interfaces. Otherwise, the ultrasound energy would be reflected back at the transducer surface and would not penetrate into the body.

When the ultrasound transducer is rotated, the probe surface should not move relative to the body to which it is coupled. This is normally achieved by integrating an acoustic window fixed on the endoscope surface. The acoustic coupling between the moving ultrasound transducer and the acoustic window is achieved by filling the intermediate volume by an acoustically appropriate liquid or grease. Care has to be taken that the volume is completely filled by the acoustic medium and that no air penetrates in that acoustic path.

US Patent No. 6,425,870 describes a transesophageal ultrasonic probe with a phased-array transducer and a motor both placed in the probe head. For ultrasonic coupling the probe head is filled with an acoustic coupling fluid. Thus the motor, mechanical gear and the entire electric circuitry is immersed in the coupling fluid. This is disadvantageous in several ways, especially with regard to the sealing of moving or rotating parts.

It is the object of the present invention to avoid these disadvantages by providing a motorized transesophageal probe with the motor located in the probe tip in which the motor, gear and electric connections are outside the acoustic fluid.

According to the present invention this is achieved by providing a liquid film between the acoustic window and the transducer and by spring means pressing the transducer against the film and the acoustic window, thereby simultaneously achieving acoustic coupling and a sliding bearing effect.

In the following a preferred embodiment of the invention is described in conjunction with the accompanying drawings.

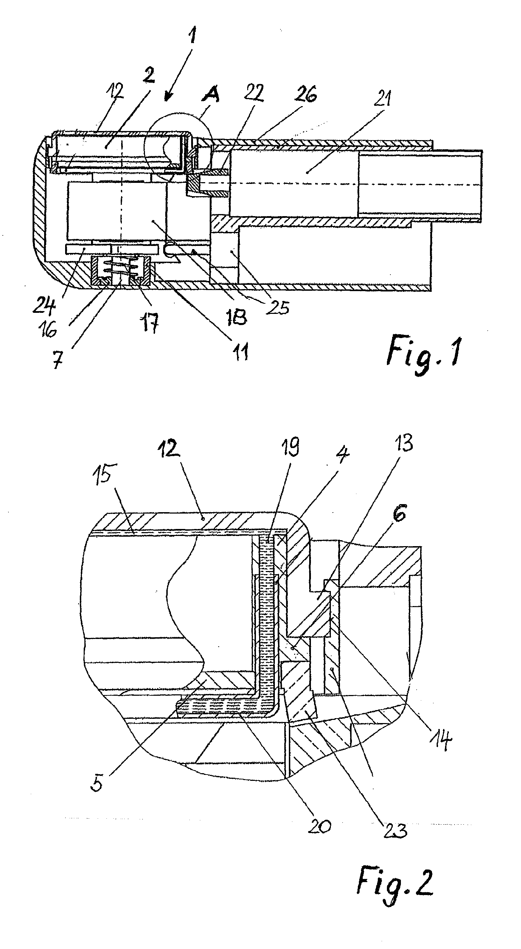

- Fig. 1: is a schematic cross-sectional view of an endoscope head
- Fig. 2: is an enlarged view of the area designated "A" in Fig. 1

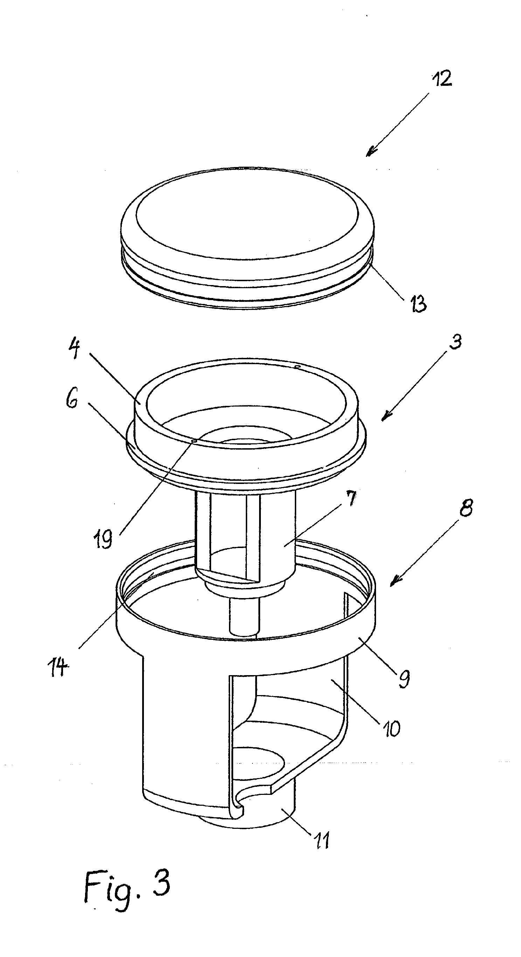

- Fig. 3: is an exploded view of the actual transducer arrangement.

As shown in the figures a transducer arrangement 1 comprises a phased array transducer 2 of flat cylindrical shape integrated in a housing 3 with a cylindrical wall 4 and a support plate 5 for accommodating the transducer. On its outer surface the cylindrical wall 4 is surrounded by an annular flange 6 with a toothed rim for interaction with a driving gear. On its rear side the support plate 5 is provided with a cylindrical shaft 7.

The transducer arrangement is rotatably mounted in a cage-shaped guiding holder 8 with an upper cylindrical portion 9, several braces 10 extending between the upper cylindrical portion and a lower pot-shaped portion 11 with an integrated bearing 16 for shaft 7 of the transducer housing 3.

An acoustic window or membrane 12 has the shape of an inverted cup with a flange 13 around its edge. Flange 13 is closely fitted into an annular groove 14 on the interior side of the upper cylindrical portion 9 of holder 8.

The phased array transducer 2 consists of sixty-four transducer elements which are electrically connected by a flexible print 18 which is wound around the shaft 7 of the transducer housing.

When assembled the upper annular surface of the flange 6 of the transducer housing 3 bears against the lower edge of the membrane 12, thus constituting a sliding bearing. Moreover the outer surface of cylindrical wall 4 is guided by the cylindrical interior surface of the membrane. The lower end of shaft 7 is guided in the sliding bearing 16 integrated in the bottom of holder 8. A spiral spring 17 between support plate 5 and the lower portion 11 of holder 8 pushes the transducer upwards against the membrane 2.

In this assembled configuration the upper surface of the transducer 2 fits exactly to the rear or inner flat surface of the membrane 12, but leaves a narrow gap 15 of about 0.1 mm. The gap 15 between the membrane 12 and the rotating transducer 2 is filled with a liquid which forms a thin liquid film. This intermediate liquid film serves as acoustic coupling medium.

The liquid is retained in gap 15 by the effect of capillary forces so that no special seal is needed. An expansion volume is provided by a narrow bore hole 19 between the upper edge of cylindrical wall 4 and the lower surface of support plate 5 and a capillary tube 20 mounted in this bore hole and extending across the lower surface of support plate 5. In the present embodiment two such expansion volumes are provided opposite each other. The expansion volumes take over the volume changes of the acoustic coupling liquid caused by temperature variations.

As the capillary effect holds the coupling fluid within the gap between the transducer and the acoustic membrane, there is no need for sealing the liquid towards the outside. The friction between the moveable transducer and the static guiding system is very small.

A micro-stepper-motor 21 with a weak torque is used to rotate the ultrasound phased array. The motor 21 is located near the ultrasound transducer. A gear-wheel 22 on the motor axis fits into a second gear wheel 23 which in turn engages with the toothed rim of transducer housing 2.

By using a stepper motor, the positional information of the ultrasound transducer can be evaluated by counting the performed steps. No additional position sensor is needed in the rotation mechanism.

A step-pulley 24 is fixed on shaft 7 and cooperates with two mechanical end switches 25. By this switching arrangement the maximum rotation angle is limited to an angle between 180° and 190°.

The entire transducer arrangement together with the motor and gear is mounted inside the tip of an endoscopic tube 26 of generally well-known kind.

Control of the motor is effected in a conventional way from the other end of endoscope 26. A micro-controller (not shown) is located in the connector of the ultrasound probe and controls the motor rotation velocity, the rotation orientation and counts the steps the motor performs. So, the controller has always track of the actual rotation angle of the ultrasound transducer. The controller checks also the status of the end-switches. If the transducer reaches one of the two end switch positions, the controller compares the evaluated actual rotation angle with the effective angle of the end position. If there is any discrepancy, the controller can signal an error in the position tracking.

A temperature sensor in the ultrasound probe at the tip of the endoscope (not shown) is also read by the micro-controller. If the evaluated temperature exceeds certain limits (e.g. 41 °C), the micro-controller disables the motor rotation and signals a corresponding message.

While the described embodiment of the invention is an transesophageal echocardiography probe, the invention is of course not limited to this embodiment. It is equally well adapted for use in any kind of multiplane ultrasound echographic exploration, such as endorectal, transvaginal, transthoracal and other medical uses as well as non-medical applications.

## Claims

1. Multiplane ultrasound probe with a probe tip containing an acoustic window, a phased array transducer and a motor for rotating the transducer array, comprising a liquid film between the acoustic window and the transducer and spring means pressing the transducer against the film and the acoustic window, thereby simultaneously achieving acoustic coupling and a sliding bearing effect.

2. A multiplane ultrasound probe according to claim 1 comprising an expansion volume connected to the liquid film.
